# EUROPÄISCHE PATENTSCHRIFT

(11) **EP 3 284 511 B1**
(45) Veröffentlichungstag und Bekanntmachung des Hinweises auf die Patenterteilung: **09.06.2021**
(21) Anmeldenummer: 16184298.4
(22) Anmeldetag: 16.08.2016
(51) Int. Cl.: A61N 1/375, A61N 1/05, A61N 1/02, A61B 5/042, A61B 5/00

(54) **ELEKTRODENLEITUNG, IMPLANTAT UND VERFAHREN ZUR IDENTIFIKATION EINER ELEKTRODENLEITUNG**
ELECTRODE LEAD, IMPLANT AND METHOD FOR THE IDENTIFICATION OF AN ELECTRODE LEAD
LIGNE D'ELECTRODES, IMPLANT ET PROCEDE D'IDENTIFICATION D'UNE LIGNE D'ELECTRODES

(43) Veröffentlichungstag der Anmeldung: 21.02.2018
(73) Patentinhaber: BIOTRONIK SE & Co. KG, 12359 Berlin (DE)
(72) Erfinder: Skerl, Olaf, 18209 Bad Doberan (DE); van Ooyen, André, 10717 Berlin (DE)
(74) Vertreter: Randoll, Sören

(56) Entgegenhaltungen:
- DE-A1-102008 040 867
- US-A- 5 720 293
- US-A1- 2007 203 547
- US-A1- 2014 343 633

## Beschreibung

Die Erfindung betrifft eine Elektrodenleitung zur Verbindung mit einem Implantat mittels eines Steckers mit mindestens einem elektrischen Leiter und einem Isolationsmantel, ein Verfahren zur Identifikation einer Elektrodenleitung, ein Implantat mit einer Buchse zur Verbindung mit dem Stecker einer Elektrodenleitung, ein Verfahren zur Konfiguration eines Systems aus Implantat und mindestens einer Elektrodenleitung sowie einen Ausleseadapter.

Implantate (implantierbare Medizingeräte, IMD) wie Herzschrittmacher (Pacemaker), Defibrillatoren oder neurologische Geräte wie Himschrittmacher für tiefe Hirnstimulation (Deep Brain Stimulation), Rückenmarkstimulationsgeräte, TENS (Transcutaneous Electrical Nerve Stimulator), Geräte für muskuläre Stimulationstherapie, oder Diagnosegeräte, welche die chemischen Eigenschaften des Blutes des Patienten, anderer Körperteile oder andere Körpereigenschaften und -parameter untersuchen, verwenden häufig Elektrodenleitungen, die in den Körper des Patienten geführt werden und dort zumindest über den Zeitraum der Behandlung oder Messung verbleiben. Die Elektrodenleitungen sind mit dem Implantat elektrisch leitend verbunden.

Die Implantate umfassen üblicherweise ein körperverträgliches Gehäuse mit einer dazugehörigen elektronischen Schaltung und einer Energieversorgung, z. B. einer Batterie. Das Gehäuse besitzt mindestens eine Buchse, an der eine oder mehrere Elektrodenleitungen angeschlossen werden können, beispielsweise mittels eines Steckers. Eine Elektrodenleitung dient der Übertragung der elektrischen Energie bzw. von Daten vom Gehäuse zu dem behandelten oder untersuchten Körperteil und umgekehrt.

Im Rahmen der vorliegenden Erfindung wird unter dem Begriff "Elektrodenleitung" eine Leitung mit mindestens einem elektrischen Leiter zusammen mit seinem umhüllenden Isolationsmantel sowie allen weiteren funktionellen Elementen, die mit der Leitung fest verbunden sind, verstanden. Die Elektrodenleitung umfasst in der Regel auch eine sogenannte Elektrodenspitze, mittels der die elektrische Energie in das zu behandelnde Gewebe eingeleitet wird. Häufig ist eine Elektrodenspitze mit Ankerelementen oder Haltestrukturen versehen, mit denen die Konstanz der räumlichen Lage der Übergangsstelle der elektrischen Energie in das zu behandelnde Gewebe sichergestellt wird. Elektrodenleitungen können auch ein oder mehrere weitere Elemente wie z. B. eine Ringelektrode oder eine Wendel umfassen, mittels derer elektrische Energie in das zu behandelnde Gewebe eingeleitet wird. Die Elektrodenspitze kann als Ableit-, Stimulations- oder Messelektrode ausgebildet sein. Weiter besitzt die Elektrodenleitung in der Regel einen Stecker, mit der die Elektrodenleitung mit einem Implantat verbunden werden kann, wobei der Stecker hierfür in eine Buchse des Implantats eingesteckt wird. Der Stecker besitzt einen oder mehrere Anschlüsse, wobei jeder Anschluss mit einem elektrischen Leiter der Elektrodenleitung verbunden ist, Wobei dieser Leiter auch aus mehreren parallel geführten, gegeneinander isulierten Leitern bestehen kann.

An moderne Implantate, beispielsweise einen Mehrkammer-Herzschrittmacher, werden häufig mehrere Elektrodenleitungen angeschlossen. Hierbei besteht das Bestreben, die Elektrodenleitungen und ihre Anschlüsse möglichst dünn bzw. klein zu gestalten. Dies erschwert andererseits die gut sichtbare Markierung der Stecker und Anschlüsse sowie die Unterscheidung der Elektrodenleitungen. Darüber hinaus steigt mit zunehmender Anzahl von Elektrodenleitungen die Gefahr, dass einzelne Elektrodenleitungen verwechselt und/oder falsch angeschlossen werden. Deshalb ist es wünschenswert, wenn ein Implantat erkennt, welche Elektrodenleitungen angeschlossen sind, so dass es diese adäquat ansteuern kann. Dazu müssen die Elektrodenleitungen und/oder ihre Eigenschaften für das Implantat identifizierbar sein.

Dokument US 2004/0073265 A1 beschreibt eine Vorrichtung, die eine Möglichkeit bietet, falsch verbundene Koronarleitungen und/oder falsche Verbindungen zu Herzrhythmusmanagement-Vorrichtungen zu erkennen. Dazu erzeugt eine Spannungseinbringungsvorrichtung eines Schrittmachers einen Spannungspuls zwischen einer Elektrode, die mit einer Leitung mit dem Schrittmacher verbunden ist, und einer Kopf- oder Gehäuseelektrode des Schrittmachers. Die Gehäuseelektrode sendet ein Verbindungssignal. Die Elektrode wird genutzt, um ein korrespondierendes Verbindungssignal unter Nutzung der Leitung zu messen. Ein Messmodul der Vorrichtung misst weiter eine oder mehrere Eigenschaften des korrespondierenden Verbindungssignals wie seine Stromstärke, Spannung, Impedanz und/oder sein Zeitverzug (nach Aussendung des Spannungspulses). Die Signaleigenschaften können von einer oder mehreren Leitungen und/oder vom übermittelnden Gewebe und Flüssigkeiten (beispielsweise einem Herz inklusive einer oder mehrere seiner Kammern), die dazwischen liegen, beeinflusst werden. Ein Vergleichsmodul des Schrittmachers kann daraufhin feststellen, ob die Leitung ordnungsgemäß zu einem Kontakt des Schrittmachers geführt ist, wobei eine oder mehrere Eigenschaften des korrespondierenden Verbindungssignals mit geeigneten vorgewählten Wertebereichen verglichen werden. Beispielsweise kann eine gemessene Impedanz mit einem erwarteten Impedanzbereich verglichen werden. Die in der Druckschrift beschriebene Vorrichtung identifiziert damit nicht selektiv die Leitung, sondern testet vielmehr, ob das korrespondierende Vergleichssignal, das über eine Leitung nach Anregung des Körpers durch einen Spannungspuls einer Gehäuseelektrode des Schrittmachers empfangen wird, Eigenschaften innerhalb eines vorgegebenen Wertebereichs aufweist. Die Eigenschaften des korrespondierenden Vergleichssignals werden auch durch das angeregte Körpergewebe zwischen der Gehäuseelektrode des Schrittmachers und der empfangenden Elektrode bestimmt. Nur sehr grobe Abweichungen, wie sie durch eine nicht verbundene oder vollkommen falsche Art von Leitung auftreten, können sicher auf die Leitung zurückgeführt werden, geringere Abweichungen können körperbedingt sein. Die oben genannte Vorrichtung kann damit die sichere und gezielte Erkennung und Unterscheidung von Elektrodenleitungen mit ähnlichen Eigenschaften nicht gewährleisten.

Eine ähnliche Vorrichtung wird auch in Dokument US 2006/0212083 A1 offenbart. Auch in dieser Druckschrift wird betont, dass die Signaleigenschaften von den Leitungen und/oder von dem/der dazwischen liegenden übermittelnden Gewebe und Flüssigkeit beeinflusst werden.

Dokument US 2011/0112609 A1 beschreibt ein System zur Rückenmarksstimulation mit mindestens einer implantierbaren Stimulationsleitung. Es umfasst insbesondere ein ärztliches Programmiergerät und einen implantierbaren Pulsgenerator, der mit einer oder mehreren implantierbaren Stimulationsleitungen verbunden ist, die je eine Vielzahl von Elektroden tragen. Die Stimulationsleitung weist ein oder zwei Leitungskörper auf. Die Elektroden passen genau in den Epiduralraum der Wirbelsäule. Da das dortige Gewebe leitend ist, können elektrische Messungen zwischen den Elektroden durchgeführt werden. Ein Kontrollschaltkreis des implantierbaren Pulsgenerators erfasst solche elektrische Messungen, so dass das ärztliche Programmiergerät automatisch die einzelnen Leitungskörper identifizieren kann, die mit dem implantierbaren Pulsgenerator verbunden sind. Die elektrischen Messungen des Kontrollschaltkreises zur Identifikation der verbundenen Leitungskörper sind Feldpotentiale. Der Kontrollschaltkreis kann auch die Impedanz an jeder Elektrode messen, um die Kopplungseffizienz zwischen der jeweiligen Elektrode und dem Gewebe zu bestimmen und um die Fehlerkennung bezüglich der Verbindung zwischen der Elektrode und dem analogen Ausgabeschaltkreis des implantierbaren Pulsgenerators zu bestimmen. Bei dem bekannten System ist von Nachteil, dass die Identifikation nicht durch den implantierbaren Pulsgenerator selbst, sondern durch ein zusätzliches ärztliches Programmiergerät erfolgt.

Dokument US 2012/0123496 A1 beleuchtet die Erkennung der Verbindung und die Identifikation des Typs einer implantierten Leitung für eine implantierte medizinische Vorrichtung. Die Vorrichtung weist einen Prozessor auf, der die Verbindung sowie den Leitungstyp der Leitung bestimmen kann. Zunächst prüft ein Signalmessmodul die Verbindung der Leitungen, indem es Werte elektrischer Parameter während eines Signals zwischen zumindest zwei Elektroden prüft, insbesondere der Impedanz. Eine oder mehrere Leitungen können darin integrierte, aktive Elektronik aufweisen, die einen oder mehrere darin integrierte modulare Schaltkreise beinhaltet, je nachdem, ob die Leitung unipolar oder multipolar ist. Jeder der modularen Schaltkreise ist in der Lage, eine Vielzahl von Elektroden der Leitung zu steuern, und schließt eine Schaltungsanordnung ein, die elektrisch mit einer oder mehreren Elektroden der Leitung verbunden ist. Als solcher wirkt jeder der modularen Schaltkreise einer Leitung als Schnittstelle zwischen der implantierten medizinischen Vorrichtung und den Elektroden, mit denen der modulare Schaltkreis verbunden ist. Für die Messung der Impedanz steuert der Prozessor der Vorrichtung den modularen Schaltkreis, sodass dieser einen Spannungspuls zwischen einer ersten und einer zweiten Elektrode liefert. Das Signalmessmodul misst den resultierenden Strom und der Prozessor leitet den Impedanzwert ab. In einem weiteren Schritt sendet der Prozessor ein Abfragesignal entlang eines ersten Leiters der Leitung, um eine Antwort von den modularen Schaltkreisen über eine zweite Leitung zu erhalten. Eine solche Antwort von jedem modularen Schaltkreis liefert dem Prozessor Informationen zu dem modularen Schaltkreis und den Elektroden, die er steuert. In einem weiteren Konfigurationsschritt sendet der Prozessor ein Signal über die erste Leitung. Der Konfigurationsschritt schließt ein, dass die aktive Konfiguration der modularen Schaltkreise programmiert wird. Bezüglich Leitungsausführungen und darin verwendeter aktiver Elektronik bzw. modularer Schaltkreise wird auf das Dokument US 7,713,194 verwiesen. Gemäß dieser Druckschrift ist der modulare Schaltkreis derart ausgestaltet, dass er durch einen Bus gesteuert wird. Die Druckschrift US 2012/0123496 A1 beschreibt demnach, dass die zusätzliche Schnittstellenelektronik modularer Schaltkreise erkannt und somit die Elektrodenleitung bestimmt werden kann. Bei der bekannten Vorrichtung ist nachteilig, dass komplexe modulare Schaltkreise mit aktiver Elektronik zur Steuerung der Elektroden implementiert und programmiert werden müssen. Ferner beziehen sich die Informationen nur auf die modularen Schaltkreise und die damit verbundenen Elektroden, nicht aber auf die Leitung als Ganzes.

Ein Verfahren und eine Vorrichtung zur automatischen Erkennung von implantierbaren medizinischen Leitungen und deren Konfiguration sind in dem Dokument US 2003/0018369 A1 dargestellt. Dafür ist ein erster Kommunikationsschaltkreis, der Daten wie Modell- und Seriennummer, technische Information und Kalibrierungsdaten speichert, mit der Leitung verbunden oder in ihr integriert. Dieser weist einen Empfänger und einen Sender auf, um Datensignale von einer externen Quelle zu empfangen. So kann er bei der Herstellung mit Identifikationsdaten, Kalibrierungsdaten und anderen Daten programmiert werden. Der erste Kommunikationsschaltkreis ist als passiver Transponder ausgeführt und weist neben dem Receiver und Transmitter weiter einen Energiekoppler zur Energieversorgung und einen Steuerschaltkreis auf, welcher mit einem nicht-flüchtigen Speicher verbunden ist. Der Steuerschaltkreis liefert die Informationen zum Transmitter/Receiver des Transponders, der die Daten via RF oder anderer Kommunikation übermittelt. Während der Implantation der Leitung oder danach können die Informationen zu einem zweiten Kommunikationsschaltkreis außerhalb der Leitung transferiert werden. Die transferierten Daten können zur Identifikation der Leitung genutzt werden, in eine Patientenakte aufgenommen und in einen Zentralspeicher für die Nutzung durch Gesundheitsdienstleister transferiert werden. Anhand des Transponders kann die Leitung automatisch erkannt werden und die im Speicher abgelegten Daten können direkt transferiert und weitergegeben werden. Der Transponder benötigt neben einem Transmitter und Receiver allerdings eine separate Energieversorgung, eine Steuereinheit und einen programmierbaren, digitalen Speicher. Dadurch ist der Gesamtaufbau der Leitung vergleichsweise aufwendig und teuer.

Auch Dokument US 2014/0343633 A1 stellt eine elektrisch identifizierbare Elektrodenleitung mit einem Identifikationsmodul, welches zumindest einen Filter, einen Stromkonverter, eine Kommunikationsschaltung, einen Lastschalter und eine Speichereinheit wie ein EPROM zur Speicherung eines Identifikationscodes aufweist. Vor der Einbringung des Implantats wird jede Leitung implantiert und mit dem implantierbaren Pulsgenerator (oder einem externen Pulsgenerator) verbunden, welcher dann selbstidentifizierende Daten vom Identifikationsmodul ausliest und diese Information an eine externe Vorrichtung wie den Arztprogrammierer übermitteln kann. Dafür kann das Identifikationsmodul bis zu 32 Byte Daten speichern. Dieses Verfahren wird für jede Leitung, die implantiert wird, wiederholt. Das Identifikationsmodul nutzt zwei vorhandene Kontakte der Leitung zur Verbindung mit dem implantierbaren Pulsgenerator. Wie im zuvor genannten Dokument wird auch für diese bekannte Elektrodenleitung ein digitaler Speicher benötigt und der Aufbau des Identifikationsmoduls ist ähnlich komplex.

Die Aufgabe der vorliegenden Erfindung besteht deshalb darin, eine Elektrodenleitung zu schaffen, die zuverlässig und eindeutig identifiziert werden kann, aber zugleich einfach aufgebaut ist und kostengünstig hergestellt werden kann. Die Aufgabe besteht ferner darin, ein einfaches Verfahren zur Identifikation einer Elektrodenleitung anzugeben, welches eine eindeutige Zuordnung eines elektrischen Leiters der Elektrodenleitung zu einem Anschluss des Implantats ermöglicht. Die Aufgabe besteht ferner darin, ein entsprechendes Implantat zu schaffen.

Die obige Aufgabe wird gelöst durch eine Elektrodenleitung mit den Merkmalen des Anspruchs 1.

Die erfindungsgemäße Elektrodenleitung besitzt einen in dem Isolationsmantel angeordneten ID-Schaltkreis mit einem ID-Chip, wobei der ID-Schaltkreis mit seinem ersten Anschluss mit einem elektrischen Leiter und mit seinem zweiten Anschluss mit einem außen auf der Elektrodenleitung freiliegenden elektrisch leitenden Element verbunden ist, wobei der ID-Chip eine Speichereinheit aufweist und das elektrisch leitende Element in Längsrichtung der Elektrodenleitung in einem vorgegebenen Abstand von dem Stecker angeordnet ist, wobei in der Speichereinheit mindestens eine individuelle Eigenschaft der Elektrodenleitung, beispielsweise die zu der Elektrodenleitung zugehörige ID-Nummer, Losnummer, Typ, Ausführung, Revision oder dergleichen gespeichert ist. Zusätzlich oder alternativ kann die mindestens eine individuelle Eigenschaft Herstellungsdaten zu der Elektrodenleitung, das Implantationsdatum und/oder -ort und/oder die MRI-Zulassung enthalten.

Der hermetisch in dem Isolationsmantel nach außen verschlossene ID-Schaltkreis, dessen ID-Chip vorzugsweise eine Eindraht-Schnittstelle aufweist, bei der sowohl die Energieversorgung als auch die Informationsübertragung über einen einzigen gemeinsamen Leiter und einen Rückleiter erfolgt, realisiert einen Signalweg über einen elektrischen Leiter der Elektrodenleitung, den ID-Schaltkreis mit ID-Chip, das außen auf der Elektrodenleitung freiliegende elektrisch leitende Element, das vorzugsweise als elektrisch leitender Ring, Draht, Seil, Litze oder Folie ausgebildet ist, und das mit dem elektrisch leitenden Element verbundene Körpergewebe zurück zum Implantat im implantierten Zustand. Hierbei ist die Verwendung des Körpergewebes als Rückleiter zum Implantatsgehäuse trotz des nicht vernachlässigbaren elektrischen Widerstands möglich, da die zum Betrieb des ID-Chips des ID-Schaltkreises notwendigen Ströme sehr gering sind und beispielsweise im Bereich von 2 µA bis 10 µA liegen. Der Aufbau des Implantats, insbesondere im Bereich der Anschlüsse, muss für die Identifikation der Elektrodenleitung nicht grundsätzlich geändert werden. Zudem ist auch der Aufbau der erfindungsgemäßen Elektrodenleitung einfach und kostengünstig. Das Material eines derartigen elektrisch leitenden Elements kann beispielsweise ein Material oder mehrere Materialien der Gruppe enthaltend Ti, Pt, Pd, Ir, Ni und Co sowie Legierungen hieraus, beispielsweise MP35N oder Pt/Ir, gegebenenfalls mit einer vorzugsweise fraktalen Beschichtung aus Ir oder TiN, aufweisen. Wichtig ist hierbei, dass die Oberfläche des elektrisch leitenden Elements, welche Kontakt zum Körpergewebe hat, mit einer Oberfläche von mindestens 1 mm² ausreichend groß ist, um einen möglichst kleinen Übergangswiderstand zu erzielen.

Die Eindraht-Schnittstelle ist grundsätzlich bekannt. Hierbei handelt es sich um eine serielle Schnittstelle für bidirektionale Kommunikation zwischen einem Master und einem Slave bei der gewöhnlich Daten asynchron und im Halbduplexverfahren übertragen werden, wobei sowohl die Energieversorgung als auch die Informationsübertragung gleichzeitig über einen einzigen gemeinsamen Leiter und einen Rückleiter stattfindet. Dabei erfolgt bei der Eindraht-Schnittstelle eine Datenübertragung auf den Zuleitungen für die Versorgungsspannung gewöhnlich mittels Modulation des versorgenden Stroms bzw. der versorgenden Spannung für Datenflüsse vom Master zum Slave und mittels Lastmodulation für Datenflüsse vom Slave zum Master. Eindraht-Schnittstellen sind zum Beispiel realisiert als 1-Wire® Schnittstelle (Dallas Semiconductor Corp.), UNI/O® Schnittstelle (Microchip Technology Inc.) oder Highway Addressable Remote Transducer als HART-Standard (Feldbus-Norm IEC 61158). Folglich werden lediglich zwei Anschlüsse am ID-Schaltkreis benötigt, welche sowohl für die Spannungsversorgung als auch für die Kommunikation mit dem ID-Chip dienen. Auch aus diesem Grund ist der Aufbau der erfindungsgemäßen Elektrodenleitung einfach. Chips mit Eindraht-Schnittstelle sind kostengünstig kommerziell verfügbar. Aufgrund des Vorteils der Eindraht-Schnittstelle, eine einzige Datenader zur Stromversorgung und als Sende- und/oder Empfangsleitung zu nutzen, kann auch eine Identifikation von Elektrodenleitungen erfolgen, welche lediglich einen einzigen elektrischen Leiter aufweisen, zum Beispiel unipolare Elektroden oder Schock-Elektroden.

Ein weiterer Vorteil der erfindungsgemäßen Elektrodenleitung besteht darin, dass der ID-Schaltkreis nicht im Signalweg zwischen dem Stecker und einem Stimulationselektrodenpol liegt. Hierdurch wird die Funktion der Elektrodenleitung als Ableit-, Stimulations- oder Messelektrode nicht beeinträchtigt, wenn der ID-Schaltkreis schadhaft sein sollte.

Bei der erfindungsgemäßen Elektrodenleitung kann ferner auf den Einsatz von RFID-Komponenten verzichtet werden. Sie stellt damit eine Alternative zu Identifizierungslösungen unter Verwendung von RFID-Techniken dar.

Die Ausbildung des elektrisch leitenden Elements als elektrisch leitender Ring, Draht, Seil, Litze oder Folie, der oder die so auf und/oder in dem Isolationsmantel angeordnet ist, dass er oder sie außen frei liegt und elektrisch kontaktiert werden kann, führt zu einer guten elektrisch leitenden Anbindung des ID-Schaltkreises an das Körpergewebe.

Es ist weiterhin von Vorteil, wenn die Speichereinheit einen beschreibbaren Speicherbereich aufweist, sodass beispielsweise Implantationsdaten, wie der Implantationsort oder das Implantationsdatum in den ID-Chip eingeschrieben werden können, sodass diese Information später weiterhin zur Verfügung steht.

Besonders bevorzugt weist der ID-Schaltkreis einen Schalttransistor auf, der parallel zu dem ID-Chip geschaltet ist und zur Kommunikation mit dem Implantat mittels Lastmodulation dient.

In einem bevorzugten Ausführungsbeispiel weist der ID-Schaltkreis parallel zu den Spannungsversorgungsanschlüssen des ID-Chips einen Spannungsbegrenzer auf, um den ID-Chip vor unzulässig hohen Spannungen, beispielsweise bei einer Schockabgabe, zu schützen. Hierfür kann beispielsweise eine Z-Diode oder ein Varistor eingesetzt werden. Zur Energieversorgung während der Lastmodulation weist der ID-Schaltkreis eine Parallelschaltung aus Diode und Pufferkondensator zur Energieversorgung auf.

Die obige Aufgabe wird ferner durch ein Verfahren zur Identifikation einer oben beschriebenen Elektrodenleitung mittels eines Implantats mit einer ID-Kommunikationseinheit umfassend die folgenden Schritte gelöst:
- Verbinden des ersten Anschlusses des ID-Schaltkreises und des zweiten Anschlusses des ID-Schaltkreises mit der ID-Kommunikationseinheit des Implantats, wobei der erste Anschluss des ID-Schaltkreises mit einem vorgegebenen elektrischen Leiter der Elektrodenleitung und der zweite Anschluss des ID-Schaltkreises mit einem Gehäuse des Implantats verbunden wird,
- Anlegen einer Wechselspannung zwischen dem ersten Anschluss und dem zweiten Anschluss des ID-Schaltkreises,
- Auslesen mindestens einer gewünschten individuellen Eigenschaft aus der Speichereinheit des ID-Chips über eine Eindraht-Schnittstelle des ID-Chips mittels der ID-Kommunikationseinheit,
- Speichern der mindestens einen ausgelesenen individuellen Eigenschaft in einer Speichereinheit des Implantats.

Das erfindungsgemäße Verfahren zeichnet sich dadurch aus, dass es einfach durchführbar ist und zu einer zuverlässigen Identifikation einer Elektrodenleitung führt. Hierbei ist der Einsatz von Wechselspannung für die Stromversorgung des ID-Chips und die Datenkommunikation mit dem Chip entscheidend für das erfindungsgemäße Verfahren. Die für die Stromversorgung und die Datenkommunikation notwendigen Spannungen liegen über der Schwelle für eine Stimulation des Gewebes. Die eingesetzte Wechselspannung (vorzugsweise Spannung U_{eff} von 2 V bis 7 V; Frequenz f von 20 kHz bis 500 kHz) verhindert eine Stimulation des Gewebes während der Ansteuerung des ID-Chips.

Das erfindungsgemäße Verfahren erlaubt es auf einfache, kostengünstige und schnelle Weise, mindestens eine individuelle Eigenschaft einer Elektrodenleitung, beispielsweise deren Seriennummer und/oder deren Typ, zu bestimmen. Der Vorteil des erfindungsgemäßen Verfahrens liegt weiter darin, dass der ID-Schaltkreis bedarfsgerecht angeregt werden kann. Das spart Energie. Der ID-Schaltkreis ist nicht auf eine dauerhafte Energieversorgung angewiesen.

Es ist ferner bevorzugt, dass durch die ID-Kommunikationseinheit über die Eindraht-Schnittstelle des ID-Chips vorgegebene Daten in einen beschreibbaren Speicherbereich der Speichereinheit des ID-Schaltkreises eingeschrieben werden. Wie oben bereits erläutert wurde, können somit auch Implantationsdaten in der Elektrodenleitung gespeichert werden.

Die Erfindung bezieht sich auch auf ein Verfahren zur Konfiguration eines Systems aus einem Implantat und mindestens einer oben beschriebenen Elektrodenleitung. Nach dem Verfahren wird zunächst die mindestens eine, mit dem Implantat verbundene Elektrodenleitung mittels eines oben beschriebenen Verfahren identifiziert und anschließend die Ansteuerung der mit der identifizierten Elektrodenleitung verbundenen Anschlüsse des Implantats und/oder die Therapieparameter entsprechend der identifizierten Elektrodenleitung konfiguriert.

Die obige Aufgabe wird ferner gelöst durch ein Implantat mit einer Buchse zur Verbindung mit dem Stecker einer Elektrodenleitung, die oben beschrieben wird. Das Implantat weist ferner ein Gehäuse und eine ID-Kommunikationseinheit auf, wobei die ID-Kommunikationseinheit zur elektrisch leitenden Verbindung mit einem vorgegebenen elektrischen Leiter einer Elektrodenleitung und dem Gehäuse des Implantats sowie zur Erzeugung einer Wechselspannung zwischen Gehäuse und dem vorgegebenen elektrischen Leiter eingerichtet ist, wobei die ID-Kommunikationseinheit weiter zum Auslesen mindestens einer gewünschten individuellen Eigenschaft der Speichereinheit des ID-Chips, mit dem sie elektrisch leitend verbunden ist, über dessen Eindraht-Schnittstelle und Speichern der mindestens einen ausgelesenen individuellen Eigenschaft in einer Speichereinheit des Implantats eingerichtet ist. Ein weiterer Vorteil der Erfindung ist, dass das abfragende Implantat keine zusätzliche Technologie zur Messung der charakteristischen Größen des ID-Schaltkreises benötigt. Vielmehr können die vorhandenen elektrischen Einrichtungen durch Modifikation der Ansteuerung zum Auslesen der mindestens einen individuellen Eigenschaft aus dem ID-Chip der Elektrodenleitung oder zum Einschreiben von Daten in den beschreibbaren Speicherbereich des ID-Chips genutzt werden. Vorzugsweise wird mit der ID-Kommunikationseinheit ein Code der individuellen Eigenschaft bestimmt.

Ein weiterer Vorteil des erfindungsgemäßen Implantats ist, dass es die Nutzung und Ansteuerung der Elektrodenleitung gegebenenfalls anhand der ermittelten mindestens einen individuellen Eigenschaft anpassen kann. Das erlaubt einen sichereren, effektiveren Einsatz des Implantats sowie der Elektrodenleitung und verbessert den Behandlungserfolg.

Erfindungsgemäß ist ferner ein Ausleseadapter mit einer Spule vorgesehen, wobei die Spule an ihrem ersten Ende mit einem vorgegebenen elektrischen Leiter der oben beschriebenen Elektrodenleitung und mit ihrem zweiten Ende mit dem elektrisch leitenden Element der Elektrodenleitung verbindbar ist. Diese Verbindung ist beispielsweise als eine Kontaktklemme ausgebildet, welche in eine in dem Ausleseadapter angeordnete Ausnehmung (Buchse) zum Einstecken des Steckers der Elektrodenleitung hineinragt. Alternative Kontaktierungsmöglichkeiten sind ebenfalls denkbar. Die Spule ist vorzugsweise durch geeignete Beschaltung mit einem Kondensator zu einem Resonanzkreis erweitert, dessen Resonanzfrequenz vorzugsweise der Arbeitsfrequenz einer vom Implantat verschiedenen RFID-Ausleseeinrichtung entspricht. Als Arbeitsfrequenz kann beispielsweise das 125 kHz-Band verwendet werden. Vor Gebrauch kann die erfindungsgemäße Elektrodenleitung zusammen mit dem Ausleseadapter im eingesteckten Zustand in einer verschlossenen sterilen Verpackung aufbewahrt werden, wobei die mindestens eine individuelle Eigenschaft über den Ausleseadapter und beispielsweise eine übliche RFID-Ausleseeinrichtung kontaktlos mittels induktiver Kopplung zwischen der Spule des Ausleseadapters und einer entsprechenden Spule der RFID-Ausleseeinrichtung ausgelesen werden kann.

Anhand der mindestens einen individuellen Eigenschaft, insbesondere einer Seriennummer, können optional weitere individuelle Eigenschaften der Elektrodenleitung ermittelt werden, beispielsweise die Art und/oder der Typ der Elektrodenleitung, die vorgesehene Verwendung, ein vorgesehener Einsatzort, der Herstellungszeitpunkt, die maximale Nutzungsdauer, eine bestimmte elektrische Eigenschaft, eine Elektrodenanzahl, die Art und/oder der Typ angeschlossener Elektroden, eine Elektrodenkonfiguration, die Anzahl therapeutischer und/oder diagnostischer Leiter, die Länge und/oder der Querschnitt therapeutischer Leiter, Informationen zur Ansteuerung oder dergleichen. Besonders bevorzugt können solche weiteren individuellen Eigenschaften aus der Speichereinrichtung des Implantats, vorzugsweise mit einer Datenbank, entnommen werden.

Die Erfindung wird nachfolgend anhand von Ausführungsbeispielen und unter Bezugnahme auf die Figuren erläutert. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbezügen.

Es zeigen schematisch:
- Fig. 1: ein Ausführungsbeispiel einer erfindungsgemäßen Elektrodenleitung in einem Längsschnitt,
- Fig. 2: das Ausführungsbeispiel gemäß Fig. 1 in einem Querschnitt,
- Fig. 3: eine Schaltskizze für einen ID-Schaltkreis der Elektrodenleitung gemäß Fig. 1,
- Fig. 4: eine Schaltskizze eines erfindungsgemäßen Implantats zum Auslesen der in Fig. 1 dargestellten Elektrodenleitung,
- Fig. 5: das steckerseitige Ende des in Fig. 1 dargestellten Ausführungsbeispiels einer Elektrodenleitung, teilweise geschnitten, und
- Fig. 6: einen erfindungsgemäßen Ausleseadapter mit eingesteckter Elektrodenleitung gemäß Fig. 1 und eine sterile Verpackung in einem Querschnitt.

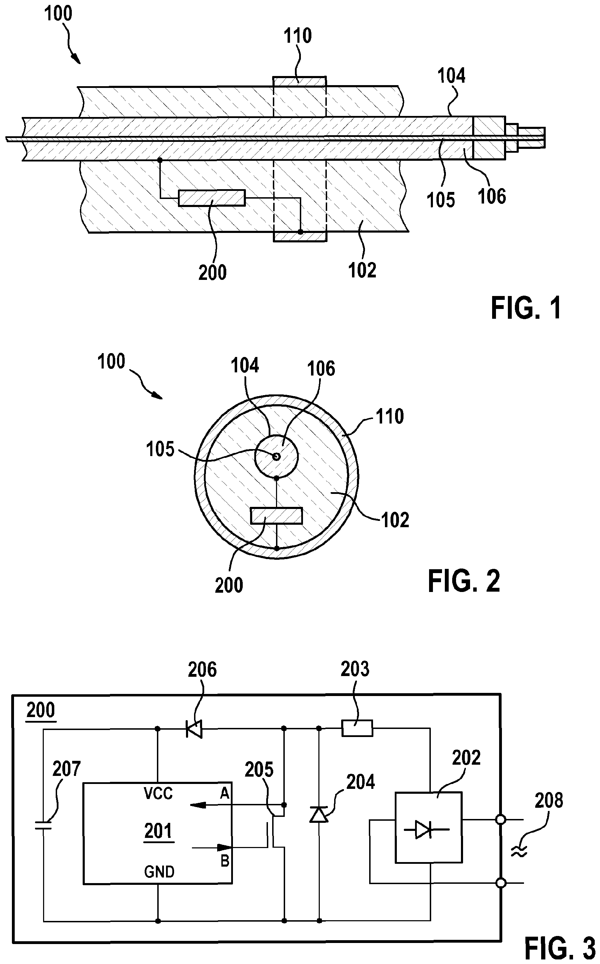

Die in Fig. 1, 2 und 5 gezeigte erfindungsgemäße Elektrodenleitung 100 mit einem außen liegenden Isolationsmantel 102 ist als Mehrlumenschlauch aus einem geeigneten extrudierbaren Polymer ausgebildet. Die Elektrodenleitung 100 weist einen Außenleiter 104 (z. B. eine drahtgewickelte Wendel oder Drahtseil), einen mit einem Kontakt an der Elektrodenspitze (z.B. die Tip-Elektrode einer Schrittmacher-Elektrode) verbundenen Innenleiter 105 sowie eine dazwischen angeordnete Isolationsschicht 106 auf. Der Außenleiter 104 ist mit einem anderen Kontakt an der Elektrodenspitze (z.B. RingElektrode einer Schrittmacherelektrode) verbunden.

Die Elektrodenleitung 100 weist ferner einen Stecker 111 auf, um die Elektrodenleitung 100 zum Beispiel mit dem Implantat 300 zu verbinden. Zur Herstellung eines Kontakts zwischen Elektrodenleitung 100 und Implantat 300 verfügt der Stecker 111 über einen Spitzenkontakt 113 und einen oder mehrere Ringkontakte 112. Der Spitzenkontakt 113 des Steckers 111 kann beispielsweise mit dem Innenleiter 105 und der Außenleiter 104 kann mit einem der Ringkontakte 112 des Steckers 111 verbunden sein.

Die elektrischen Leiter der Elektrodenleitung können jedoch auch als Nicht-Koaxialkabel ausgebildet sein und nebeneinander in dem Isolationsmantel 102 vorliegen. Alternativ kann lediglich ein einziger Leiter in der Elektrodenleitung 100 angeordnet sein (z.B. für eine unipolare Elektrode oder Schock-Elektrode).

Die Elektrodenleitung weist ferner, eingebettet in den Isolationsmantel 102 einen ID-Schaltkreis 200 auf. Ein erster Anschluss der Anschlüsse 208 des ID-Schaltkreises 200 ist elektrisch leitend mit dem Außenleiter 104 und ein zweiter Anschluss der Anschlüsse 208 des ID-Schaltkreises 200 mit einem außen auf dem Isolationsmantel 102 angeordneten elektrisch leitenden Ring 110 verbunden. Die Verbindung mit dem Außenleiter 104 ist vorteilhaft, da sie kostengünstig realisiert werden kann. Würde stattdessen eine Verbindung des ID-Schaltkreises 200 mit dem Innenleiter 105 erfolgen, was prinzipiell auch möglich ist, müsste die Anschlussleitung isoliert durch den Außenleiter 104 hindurch geführt werden. Durch den elektrisch leitenden Ring 110 erfolgt der Rückkontakt des ID-Schaltkreises 200 über das Körpergewebe zu dem Gehäuse eines Implantats 300, mit dem, wie unten dargestellt, mindestens eine individuelle Eigenschaft der Elektrodenleitung 100 ausgelesen werden kann. Der elektrisch leitende Ring 110 ist deshalb in einem vorgegebenen Abstand von dem Stecker, der zum Einführen in eine Buchse des Implantats 300 ausgebildet ist, um die Elektrodenleitung 100 mit dem Implantat 300 zu verbinden, angeordnet. Bei nebeneinander in dem Isolationsmantel 102 liegenden elektrischen Leitern kann der leiterseitige Anschluss 208 des ID-Schaltkreises 200 mit einem der elektrischen Leiter verbunden sein.

Das Auslesen des ID-Schaltkreises 200 erfolgt mittels Wechselspannung. Daher ist am Anschluss 208 des ID-Schaltkreises 200 ein Gleichrichter 202 vorgesehen. Die Ausgänge dieses Gleichrichters sind sowohl mit Eingängen zur Spannungsversorgung (V_{CC}, GND) eines ID-Chips 201 als auch mit dessen Signaleingängen A und B verbunden. Die Verbindung erfolgt über einen Schutzwiderstand 203, der beispielsweise einen Widerstand von 50 kΩ besitzt. Parallel zu den Eingängen zur Spannungsversorgung ist ein Spannungsbegrenzer 204, beispielsweise eine Z-Diode oder ein Varistor vorgesehen. Der Effektivwert der Wechselspannung beträgt beispielsweise 3 V mit einer Frequenz von 100 kHz. Der Gleichrichter hat eine Spannungsfestigkeit, welche der im bestimmungsgemäßen Therapiebetrieb maximal auftretenden Spannung entspricht, beispielsweise 1 kV. Über die Anschlüsse 208 für die Energieversorgung erfolgt gleichzeitig die Kommunikation mit den Signaleingängen A, B des ID-Chips 201, vorzugsweise mittels Lastmodulation durch einen Schalttransistor 205. Um die Energieversorgung des ID-Chips 201 während der Lastmodulation zu sichern, sind die Anschlüsse 208 für die Energieversorgung mit einer Schaltdiode 206 und einem Pufferkondensator 207 beschaltet. Der Pufferkondensator hat beispielsweise eine Kapazität von 1 nF.

Der ID-Chip 201 besitzt eine Eindraht-Schnittstelle zur Kommunikation mit einer ID-Kommunikationseinheit 303 des Implantats 300 zum Auslesen mindestens einer individuellen Eigenschaft (z.B. Seriennummer, Typbezeichnung etc. siehe oben) der Elektrodenleitung 100, welche in einer Speichereinheit des ID-Chips 201 gespeichert ist. Vorzugsweise weist die Speichereinheit des ID-Chips auch einen beschreibbaren Speicherbereich zum Einschreiben von Daten auf, welche über die Eindraht-Schnittstelle des ID-Chips 201 übermittelt werden können.

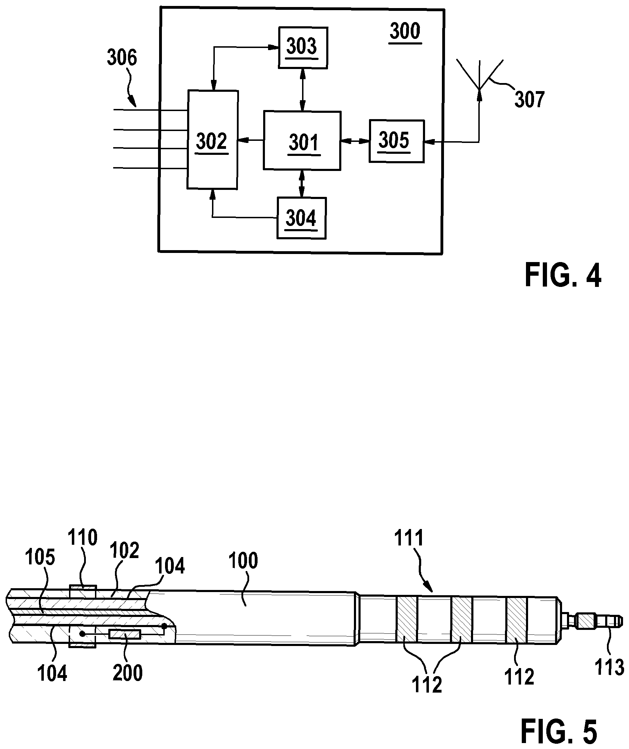

Zur Abfrage einer Elektrodenleitung gemäß Figur 1 und 2 kann beispielsweise das in Figur 4 dargestellte Implantat 300 verwendet werden. Das Implantat besitzt eine Steuerungseinheit 301, welche mit einem geeigneten Multiplexer 302 und einer ID-Kommunikationseinheit 303 verbunden ist. Ferner sind eine Therapie- und Diagnostikeinheit 304 sowie eine Telemetrieeinheit 305 vorgesehen, wobei die Telemetrieeinheit 305 mit einer Antenne 307 verbunden ist. Sowohl die Telemetrieeinheit 305 als auch die Therapie- und Diagnostikeinheit 304 sind ebenfalls mit der Steuerungseinheit 301 verbunden. Für ein Auslesen einer mit dem Implantat 300 verbundenen Elektrodenleitung 100, beispielsweise durch Einstecken des Steckers der Elektrodenleitung 100 in eine Buchse des Implantats 300, werden die entsprechenden Elektrodenanschlüsse 306 des Implantats durch den Multiplexer 302 mit der ID-Kommunikationseinheit 303 verbunden. Im Therapie- oder Diagnosebetrieb des Implantats 300 verbindet der Multiplexer 302 die Elektrodenanschlüsse 306 stattdessen mit der Therapie- und Diagnostikeinheit 304 des Implantats 300.

Für die Kommunikation mit der Eindraht-Schnittstelle des ID-Chips 201 des ID-Schaltkreises werden ein erster vorgegebener Anschluss des Implantats 100 mittels des Multiplexers 302 mit der Außenleitung 104 der Elektrodenleitung 100 und ein zweiter vorgegebener Anschluss mit dem Gehäuse des Implantats 300 verbunden. Ferner wird die ID-Kommunikationseinheit 303 aktiviert. Nun veranlasst die Steuerungseinheit 301 ein Anlegen einer effektiven Wechselspannung von beispielsweise 3 V mit einer Frequenz von 100 kHz zwischen dem vorgegebenen ersten und zweiten Anschluss, welche somit auch an den Anschlüssen 208 des ID-Schaltkreises 200 anliegt. Nun erfolgt durch die ID-Kommunikationseinheit 303 ein Auslesen mindestens einer individuellen Eigenschaft aus der Speichereinheit des ID-Chips 201 über die Eindraht-Schnittstelle und/oder ein Einschreiben von Daten in den beschreibbaren Speicherbereich des ID-Chips 201 über diese Schnittstelle. Die ausgelesenen Daten können in einer Speichereinheit der Steuerungseinheit 301 abgespeichert oder über die Telemetrieeinheit 305 an ein weiteres Gerät, beispielsweise ein externes Patientengerät, übertragen werden. Das Patientengerät kann diese Information an eine Telemonitoring-Plattform übertragen. Ebenso ist es möglich, auf diesem Wege Informationen in einem beschreibbaren Speicherbereich der Speichereinheit des ID-Chips 201 abzuspeichern, die aus der Steuerungseinheit 301 des Implantats 300 selbst, aus einem anderen Gerät (zum Beispiel Patientengerät) oder aus einer Telemonitoring-Plattform stammen.

Anhand der aus der Elektrodenleitung 100 ausgelesenen Informationen kann das Implantat 300 automatisch die Anschlussbelegung der Elektrodenleitung feststellen und konfigurieren und/oder Therapieparameter an die erkannte Elektrodenleitung anpassen. Die aus der Elektrodenleitung ausgelesenen Informationen, beispielsweise eine Seriennummer, können für die Dokumentation, die Rückverfolgbarkeit des Produktes (Chargenrückverfolgung), Fehleranalyse oder deren Auswahl für bestimmte Anwendungen verwendet werden.

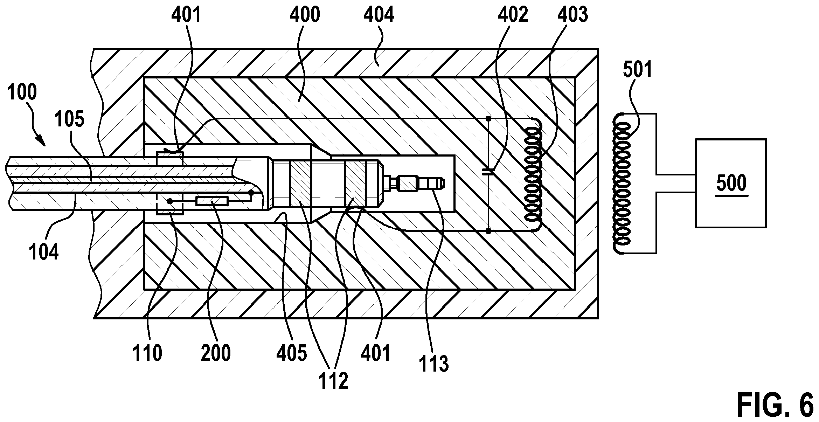

Um die Informationen aus der Elektrodenleitung auch im nicht implantierten, z.B. verpackten Zustand auslesen zu können, kann während Herstellung, Lagerung und Versand ein in Fig. 6 dargestellter Ausleseadapter 400, der eine Spule 403 aus elektrisch leitendem Material aufweist, derart auf den Stecker 111 der Elektrodenleitung 100 gesteckt werden, dass dessen einer Anschluss (Kontaktklemme 401) mit dem elektrisch leitenden Ring 110 und dessen anderer Anschluss (Kontaktklemme 401) mit einem elektrischen Leiter der Elektrodenleitung 100, zum Beispiel mit dem Außenleiter 104 über einen Ringkontakt 112 des Steckers 111, verbunden ist. Der Ausleseadapter 400 besteht vorzugsweise aus einem elektrisch nicht leitenden, nichtmetallischen Material, beispielsweise Kunststoff, Epoxidharz, Silikon, oder dergleichen. Der Ausleseadapter 400 kann z.B. zylinderförmig oder quaderförmig gestaltet sein, wobei beliebige andere Formen ebenfalls denkbar sind. Der Ausleseadapter enthält eine z.B. zylinderförmige Ausnehmung (Buchse) 405, die zum Einstecken des Steckers 111 der Elektrodenleitung dient. Aus der Innenwand der Ausnehmung 405 ragen die Kontaktklemmen 401 heraus und in die Ausnehmung 405 hinein. Die Spule 403 ist durch geeignete Beschaltung mit einem Kondensator 402 zu einem Resonanzkreis erweitert, dessen Resonanzfrequenz vorzugsweise der Arbeitsfrequenz einer vom Implantat verschiedenen Ausleseeinrichtung 500 entspricht. Vor Verwendung des Elektrodenleiters 100 ist die Kombination aus eingestecktem Elektrodenleiter 100 und Ausleseadapter 400 in einer verschlossenen sterilen Verpackung 404 angeordnet. Die sterile Verpackung 404 besteht ebenfalls vorzugsweise aus einem elektrisch nicht leitenden, nichtmetallischen Material.

In dem Zustand, in dem der Ausleseadapter auf die zu vermessende Elektrodenleitung aufgesteckt ist, ähnelt diese Anordnung in ihrer Arbeitsweise einem RFID-Transponder bekannter Art, beispielsweise einem RFID-Transponder für das 125 kHz Band. Als Ausleseeinrichtung für das Auslesen und Beschreiben der Speichereinheit des ID-Chips 201 in dieser Anordnung kann eine bekannte Ausleseeinrichtung 500 für einen solchen RFID-Transponder mit einer Abfragespule 501 verwendet werden. Mit dieser Anordnung ist es möglich, die Elektrodenleitung 100 kontaktlos mittels induktiver Kopplung auch durch eine sterile Verpackung hindurch auszulesen oder einzuschreiben. Das Auslesegerät kann ein Display enthalten, auf dem die ausgelesenen Informationen dargestellt werden können und/oder eine Eingabemöglichkeit enthalten, über die die einzuspeichernden Informationen eingegeben werden können. Fernerhin kann das Auslesegerät 500 mit einer Datenverbindung geeigneter Art an ein Datennetzwerk angeschlossen sein, in das die ausgelesenen Informationen übertragen werden oder aus dem die einzuspeichernden Informationen bezogen werden können.

### Bezugszeichenliste

- 100: Elektrodenleitung
- 102: Isolationsmantel
- 104: Außenleiter
- 105: Innenleiter
- 106: Isolationsschicht
- 110: elektrisch leitender Ring
- 111: Stecker
- 112: Ringkontakt des Steckers 111
- 113: Spitzenkontakt des Steckers 111
- 200: ID-Schaltkreis
- 201: ID-Chip
- 202: Gleichrichter
- 203: Schutzwiderstand
- 204: Spannungsbegrenzer
- 205: Schalttransistor
- 206: Diode
- 207: Pufferkondensator
- 208: Anschluss des ID-Schaltkreises 200 zur Spannungsversorgung
- 300: Implantat
- 301: Steuerungseinheit
- 302: Anschluss-Multiplexer
- 303: ID-Kommunikationseinheit
- 304: Therapie- oder Diagnostikeinheit
- 305: Telemetrieeinheit
- 306: Anschlüsse des Implantats
- 307: Antenne
- 400: Ausleseadapter
- 401: Kontaktklemme
- 402: Kondensator des Ausleseadapters 400
- 403: Spule des Ausleseadapters 400
- 404: sterile Verpackung
- 405: Ausnehmung in dem Ausleseadapter 400
- 500: RFID-Ausleseeinrichtung
- 501: Abfragespule der RFID-Ausleseeinrichtung 500

## Patentansprüche

1. Elektrodenleitung (100) zur Verbindung mit einem Implantat (300) mittels eines Steckers mit mindestens einem elektrischen Leiter (104, 105) und einem Isolationsmantel (102), umfassend einen in dem Isolationsmantel (102) angeordneten ID-Schaltkreis (200) mit einem ID-Chip (201), wobei der ID-Schaltkreis (200) mit seinem ersten Anschluss mit einem elektrischen Leiter (104) verbunden ist, wobei der ID-Chip (201) eine Speichereinheit aufweist, wobei in der Speichereinheit mindestens eine individuelle Eigenschaft der Elektrodenleitung (100), beispielsweise die zugehörige ID-Nummer, Losnummer, Typ, die Ausführung, Revision oder dergl., gespeichert ist,
**dadurch gekennzeichnet, dass** der ID-Schaltkreis (200) mit seinem zweiten Anschluss mit einem außen auf der Elektrodenleitung frei liegenden elektrisch leitenden Element (110) verbunden ist und das elektrisch leitende Element (110) in Längsrichtung der Elektrodenleitung (110) in einem vorgegebenen Abstand von dem Stecker angeordnet ist.

2. Elektrodenleitung (100) nach Anspruch 1, **dadurch gekennzeichnet, dass** der ID-Chip (201) eine Eindraht-Schnittstelle aufweist.

3. Elektrodenleitung (100) nach einem der vorhergehenden Ansprüche, **dadurch gekennzeichnet, dass** das elektrisch leitende Element als elektrisch leitender Ring, Draht, Seil, Litze oder Folie (110) ausgebildet ist.

4. Elektrodenleitung (100) nach einem der vorhergehenden Ansprüche, **dadurch gekennzeichnet, dass** die Speichereinheit einen beschreibbaren Speicherbereich aufweist.

5. Elektrodenleitung (100) nach einem der vorhergehenden Ansprüche, **dadurch gekennzeichnet, dass** der ID-Schaltkreis (200) einen Schalttransistor (205) aufweist, der parallel zu dem ID-Chip (201) geschaltet ist und zur Kommunikation mit dem Implantat (300) mittels Lastmodulation dient.

6. Elektrodenleitung (100) nach einem der vorhergehenden Ansprüche, **dadurch gekennzeichnet, dass** der ID-Schaltkreis (200) einen Spannungsbegrenzer (204) aufweist.

7. Elektrodenleitung (100) nach einem der vorhergehenden Ansprüche, **dadurch gekennzeichnet, dass** der ID-Schaltkreis eine Beschaltung aus Diode (206) und Pufferkondensator (207) zur Energieversorgung während der Lastmodulation aufweist.

8. Verfahren zur Identifikation einer Elektrodenleitung (100) nach einem der Ansprüche 1 bis 7 mittels eines Implantats (300), umfassend die folgenden Schritte:
- Verbinden des ersten Anschlusses des ID-Schaltkreises (200) und des zweiten Anschlusses des ID-Schaltkreises (200) mit einer ID-Kommunikationseinheit (303) des Implantats (300), wobei der erste Anschluss des ID-Schaltkreises mit einem vorgegebenen elektrischen Leiter (104) der Elektrodenleitung (100) und der zweite Anschluss des ID-Schaltkreises mit einem Gehäuse des Implantats (300) verbunden wird,
- Anlegen einer Wechselspannung zwischen dem ersten Anschluss und dem zweiten Anschluss (208) des ID-Schaltkreises (200),
- Auslesen mindestens einer gewünschten individuellen Eigenschaft aus der Speichereinheit des ID-Chips (201) über eine Eindraht-Schnittstelle des ID-Chips (201) mittels der ID-Kommunikationseinheit (303),
- Speichern der mindestens einen ausgelesenen individuellen Eigenschaft in einer Speichereinheit des Implantats (300).

9. Verfahren nach Anspruch 8, **dadurch gekennzeichnet, dass** durch die ID-Kommunikationseinheit (303) über die Eindraht-Schnittstelle zusätzlich vorgegebene Daten in einen beschreibbaren Speicherbereich der Speichereinheit des ID-Schaltkreises (200) eingeschrieben werden.

10. Verfahren zur Konfiguration eines Systems bestehend aus einem Implantat (300) und mindestens einer Elektrodenleitung (100) nach einem der Ansprüche 1 bis 7, umfassend die folgenden Schritte:
- Identifikation der mindestens einen, mit dem Implantat (300) verbundenen Elektrodenleitung (100) mittels eines Verfahrens nach einem der Ansprüche 8 bis 9,
- Konfiguration der Ansteuerung der mit der ausgelesenen Elektrodenleitung (100) verbundenen Anschlüsse (306) des Implantats (300) und/oder der Therapieparameter.

11. Implantat (300) mit einer Buchse zur Verbindung mit dem Stecker einer Elektrodenleitung (100) nach einem der Ansprüche 1 bis 7 und mit einem Gehäuse, mit einer ID-Kommunikationseinheit (303), welche mit einem vorgegebenen elektrischen Leiter (104, 105) der Elektrodenleitung (100) und dem Gehäuse des Implantats (300) verbindbar und zur Erzeugung einer Wechselspannung zwischen Gehäuse und dem vorgegebenen elektrischen Leiter (104) eingerichtet ist, wobei die ID-Kommunikationseinheit weiter zum Auslesen mindestens einer gewünschten individuellen Eigenschaft der Speichereinheit des ID-Chips (201), vorzugsweise über eine Eindraht-Schnittstelle des ID-Chips (201), und Speichern der mindestens einen ausgelesenen individuellen Eigenschaft in einer Speichereinheit des Implantats (300) eingerichtet ist.

## Claims

1. An electrode lead (100), intended for connection to an implant (300) by means of a plug, with at least one electrical conductor (104, 105) and an insulation sleeve (102), comprising an ID circuit (200) which is arranged in the insulation sleeve (102) and has an ID chip (201), wherein the ID circuit (200) is connected by means of its first connector to an electrical conductor (104), wherein the ID chip (201) has a memory unit, wherein at least one individual property of the electrode lead (100), for example the associated ID number, batch number, type, the design, revision or the like, is stored in the memory unit, **characterised in that** the ID circuit (200) is connected by means of its second connector to an electrically conductive element (110) exposed externally on the electrode lead and the electrically conductive element (110) is arranged at a predefined distance from the plug in the longitudinal direction of the electrode lead (110).

2. The electrode lead (100) according to claim 1, **characterised in that** the ID chip (201) has a single-wire interface.

3. The electrode lead (100) according to one of the preceding claims, **characterised in that** the electrically conductive element is designed as an electrically conductive ring, wire, cable, stranded wire or foil (110).

4. The electrode lead (100) according to one of the preceding claims, **characterised in that** the memory unit has a writeable memory region.

5. The electrode lead (100) according to one of the preceding claims, **characterised in that** the ID circuit (200) has a switching transistor (205) which is connected parallel to the ID chip (201) and is used for communication with the implant (300) by means of load modulation.

6. The electrode lead (100) according to one of the preceding claims, **characterised in that** the ID circuit (200) has a voltage limiter (204).

7. The electrode lead (100) according to one of the preceding claims, **characterised in that** the ID circuit has a circuitry formed of diode (206) and buffer capacitor (207) for power supply during the load modulation.

8. A method for identifying an electrode lead (100) according to one of claims 1 to 7 by means of an implant (300), comprising the following steps:
- connecting the first connector of the ID circuit (200) and the second connector of the ID circuit (200) to an ID communication unit (303) of the implant (300), wherein the first connector of the ID circuit is connected to a predefined electrical conductor (104) of the electrode lead (100) and the second connector of the ID circuit is connected to a housing of the implant (300),
- applying an alternating voltage between the first connector and the second connector (208) of the ID circuit (200),
- reading out at least one desired individual property from the memory unit of the ID chip (201) via a single-wire interface of the ID chip (201) by means of the ID communication unit (303),
- storing the at least one read-out individual property in a memory unit of the implant (300).

9. The method according to claim 8, **characterised in that** predefined data are additionally written into a writeable memory region of the memory unit of the ID circuit (200) by the ID communication unit (303) via the single-wire interface.

10. A method for configuring a system consisting of an implant (300) and at least one electrode lead (100) according to one of claims 1 to 7, comprising the following steps:
- identifying the at least one electrode lead (100) connected to the implant (300) by means of a method according to one of claims 8 to 9,
- configuring the actuation of the connectors (306) of the implant (300) connected to the read-out electrode lead (100) and/or the therapy parameters.

11. An implant (300) having a socket for connection to the plug of an electrode lead (100) according to one of claims 1 to 7 and having a housing, with an ID communication unit (303) which is connectable to a predefined electrical conductor (104, 105) of the electrode lead (100) and to the housing of the implant (300) and is designed to generate an alternating voltage between housing and the predefined electrical conductor (104), wherein the ID communication unit is also designed to read out at least one desired individual property of the memory unit of the ID chip (201), preferably via a single-wire interface of the ID chip (201), and to store the at least one read-out individual property in a memory unit of the implant (300).

## Revendications

1. Ligne d'électrode (100) permettant la connexion avec un implant (300) au moyen d'une fiche, dotée d'au moins un conducteur (104, 105) électrique et d'une gaine isolante (102), comprenant un circuit ID (200) disposé dans la gaine isolante (102) avec une puce ID (201), où le circuit ID (200) est relié avec un conducteur (104) électrique avec son premier connecteur, où la puce ID (201) présente une unité de mémoire, où au moins une propriété individuelle de la ligne d'électrode (100), par exemple, le numéro d'ID correspondant, le numéro de lot, le type, le modèle, la révision ou similaire, est enregistrée dans l'unité de mémoire,
**caractérisée en ce que** le circuit ID (200) est relié avec son deuxième connecteur avec un élément conducteur électriquement (110) situé à l'état libre à l'extérieur sur la ligne d'électrode et l'élément conducteur électriquement (110) est disposé à une distance prédéfinie du connecteur dans la direction longitudinale de la ligne d'électrode (110).

2. Ligne d'électrode (100) selon la revendication 1, **caractérisée en ce que** la puce ID (201) présente une interface monofilaire.

3. Ligne d'électrode (100) selon l'une des revendications précédentes, **caractérisée en ce que** l'élément conducteur électriquement est conçu sous forme d'anneau, de fil, de corde, de brin ou de film électriquement conducteur (110).

4. Ligne d'électrode (100) selon l'une des revendications précédentes, **caractérisée en ce que** l'unité de mémoire présente une zone de mémorisation pouvant être écrite.

5. Ligne d'électrode (100) selon l'une des revendications précédentes, **caractérisée en ce que** le circuit ID (200) présente un transistor de commutation (205) qui est branché en parallèle par rapport à la puce ID (201) et sert à la communication avec l'implant (300) au moyen d'une modulation de charge.

6. Ligne d'électrode (100) selon l'une des revendications précédentes, **caractérisée en ce que** le circuit ID (200) présente un limiteur de tension (204).

7. Ligne d'électrode (100) selon l'une des revendications précédentes, **caractérisée en ce que** le circuit ID présente un circuit à base d'une diode (206) et d'un condensateur tampon (207) pour l'alimentation en énergie pendant la modulation de charge.

8. Procédé d'identification d'une ligne d'électrode (100) selon l'une des revendications 1 à 7 au moyen d'un implant (300), comprenant les étapes suivantes :
- la connexion du premier connecteur du circuit ID (200) et du deuxième connecteur du circuit ID (200) avec une unité de communication ID (303) de l'implant (300), où le premier connecteur du circuit ID est relié avec un conducteur électrique (104) prédéfini de la ligne d'électrode (100) et le deuxième connecteur du circuit ID est relié avec un boitier de l'implant (300),
- l'application d'une tension alternative entre le premier connecteur et le deuxième connecteur (208) du circuit ID (200),
- la lecture d'au moins une propriété individuelle souhaitée dans l'unité de mémoire de la puce ID (201) par le biais d'une interface monofilaire de la puce ID (201) au moyen de l'unité de communication ID (303),
- le stockage de l'au moins une propriété individuelle lue dans une unité de mémoire de l'implant (300).

9. Procédé selon la revendication 8, **caractérisé en ce que** des données prédéfinies supplémentaires sont écrites dans une zone de mémorisation de l'unité de mémoire du circuit ID (200) par l'unité de communication ID (303) par le biais de l'interface monofilaire.

10. Procédé de configuration d'un système constitué d'un implant (300) et d'au moins une ligne d'électrode (100) selon l'une des revendications 1 à 7, comprenant les étapes suivantes :
- l'identification de l'au moins une ligne d'électrode (100) reliée avec l'implant (300) au moyen d'un procédé selon l'une des revendications 8 à 9,
- la configuration de l'activation des connecteurs (306) reliés avec la ligne d'électrode (100) lue de l'implant (300) et/ou de paramètres de thérapie.

11. Implant (300) avec une douille pour la connexion avec la fiche d'une ligne d'électrode (100) selon l'une des revendications 1 à 7 et avec un boitier, avec une unité de communication ID (303), laquelle peut être connectée avec un conducteur électrique (104, 105) prédéfini de la ligne d'électrode (100) et avec le boitier de l'implant (300), et est conçue pour la création d'une tension alternative entre le boitier et le conducteur électrique (104) prédéfini, où l'unité de communication ID est conçue en outre pour la lecture d'au moins une propriété individuelle souhaitée de l'unité de mémoire de la puce ID (201), de préférence par le biais d'une interface monofilaire de la puce ID (201), et pour la mise en mémoire de l'au moins une propriété individuelle lue dans une unité de mémoire de l'implant (300).
